# EUROPEAN PATENT SPECIFICATION

(11) **EP 2 967 305 B1**
(45) Date of publication and mention of the grant of the patent: **08.04.2020**
(21) Application number: 14727898.0
(22) Date of filing: 11.03.2014
(51) Int. Cl.: A61B 1/253

(54) **CLEANING A MEDICAL OR DENTAL MIRROR WITH HANDLE BY MEANS OF COMPRESSED AIR FLOW**
REINIGUNG EINES MEDIZINISCHEN ODER ZAHNÄRZTLICHEN SPIEGELS MITTELS DRUCKLUFT
NETTOYAGE D'UN MIROIR À POIGNÉE POUR LA MÉDECINE OU L'ART DENTAIRE À L'AIDE D'UN FLUX D'AIR COMPRIMÉ

(30) Priority: 12.03.2013 NL 1040090
(43) Date of publication of application: 20.01.2016
(73) Proprietor: Den Hartog, Maarten Antoon Nicolaas, 6822 ER Arnhem (NL)
(72) Inventor: Den Hartog, Maarten Antoon Nicolaas, 6822 ER Arnhem (NL)
(86) International application number: PCT/IB2014/000661
(87) International publication number: WO 2014/140795

(56) References cited:
- EP-A1- 1 972 261
- CH-A5- 594 397
- DE-A1- 3 801 613
- US-A- 5 449 290
- US-A1- 2003 076 605

## Description

Problem during the use of in particular mirrors for medical or dental examination is that these mirrors are covered by dirt and moisture, causing a significant reduction of the sharp image reflection or even making impossible any image reflection at all. This problem is described in various patent applications.

In the prior art, a method is known in which a mirror surface is cleaned just by an air flow. This can be achieved, as evidenced by US 2005/0282103 and US 005449290 through a more simple instrument. In practice, however, this method is insufficient, because the majority of the dirt is sticking to the surface and the air flow has insufficient control over it. Examples of this are US 544290 and US2005/0282103. Known are also solutions, where only air is extracted at the periphery of the mirror surface in order to remove moisture and dirt from the reflecting surface. For example, see in US 8133052. This solution does not work sufficiently either for the same reason as in the sole air flow over a surface.

The many technical solutions that have been put forward for this problem in all cases lead to the situation that the mirror or the related elongate handle increase in size and therefore provide less freedom of movement and which are more difficult to clean or sterilize, and more difficult to produce and therefore are more expensive.

Examples of this are U.S. 2004/0076019 and EP 2181643 in which a rotating mirror with an extraction system is described; U.S. 3969824, in which a fluid supply and an extraction system is described, as well as the suggestion is made to create a hydrophilic surface by means of an additive; U.S. 2010/0261132 in which a combination of compression of air and spraying liquid over the reflecting surface is described, preferably by adding of an additional substance to the liquid, through which the hydrophobic effect on the mirror reduces in order for the fluid to more easily glide over the surface.

The most effective method proves to be to render the surface extremely hydrophobic in nature. This method is known from patent application EP 1972261 which describes the keeping clean of a mirror laced in a video-endoscope by means of air projected onto the reflecting surface with a hydrophobic nature. This instrument too is sizable and difficult to sterilize.

It is the goal of the invention to come to a better sterilizable and more easily handable composition of a handle and mirror for medical or dental examination with an integrated air channel that is to be connected to the compressed air supply, and specially shaped in such a way that the mirror reflective surface is kept clean my the air flow manipulated for that purpose by this air channel. The invention thereto is offering a mirror of the aforementioned type, that is
characterized by a protrusion that is inextricably attached to the mirror on the outflow side but at the inside of the air duct and which can be inserted detachably into the handle by clamping and whereby being shaped three dimensionally in such a way that the protrusion directly narrows the opening of the air outlet in such a way that the air flow will diverge just before the outflow opening to a downward effective flow on the mirror surface whereby the protrusion on itself from all sides is accessible for cleaning and the air duct in itself stays completely hollow.

Moreover this makes possible that several mirrors of a distinctive shape, dispersion angle and size of surface can be placed in the handle with maintenance of the characteristics of the invention, by means of the three dimensional protrusion that is inextricably attached to each specific mirror.

For the purpose of the target the invention further provides a combined improvement of effect of both the air flow and the functionality of the mirror, by using a triangular shaped mirror, and the attachment of the protrusion to the handle in an angled position, and by steering the air flow from above this mounting point through an in or by the hollow handle created air duct whereby the triangular mirror shape also offers the possibility to place instruments like i.e. a drill, dental probe or tweezers next to the side of the mirror and then are observably reflected in the rear portion of the triangular reflecting surface.

In order to facilitate the cleaning and sterilization of the handle and to make possible use of the mirror with handle without air supply, the link between air supply and the handle is arranged in such a way that the air supply pipe contains a rigid end part that only is to be inserted into the air duct at the back of the handle to enable coupling through the mutual magnetically attracted rings of which one is inextricably attached along the rigid end part of the air supply and the other is inextricably attached onto the hollow handle, thus providing an air-tight connection and by which the air duct in the handle and the air supply tube are leak free connected with each other and by which the magnetic adhesive force is chosen in such a way that the coupling is maintained when a pulling force is applied in a situation that the handle is normally used and that is disconnected by means of a larger exertion hand force applied to it.

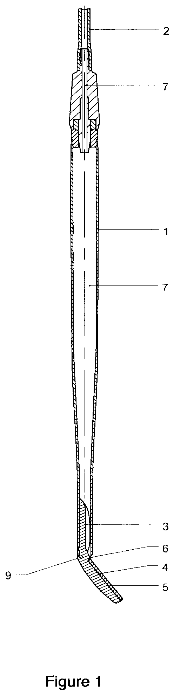

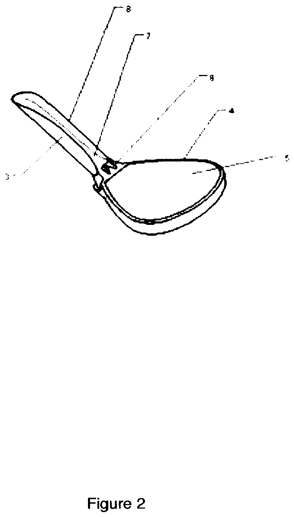

On the basis of Figures 1-5 , the invention will in a preferred embodiment be described in detail. Figure 1 shows a handle (1) in longitudinal crosssection with an air duct (7) and a flexible air supply pipe connected at the top (2) and inserted profusion at the bottom (3) connected with a mirror (4) with a hydrophobic and non-stick made reflecting surface (5) that is unlimited from the air outlet (6) of the air duct (7) to all directions of the reflecting surface (5) i.e. not limited by an upstanding casing or rim. Figure 2 shows the protrusion (3) standing on its own with the mirror (4) that is rigidly attached to it. At the upper side of the protrusion (3) either vertical walls or control surfaces (8) are formed, that have been arranged into such an angle with respect to the longitudinal direction (i.e. in the air flow direction) of the protrusion (3) and in such way that the air flowing along those surfaces is diverged congruent to the at the protrusion connected angle of the mirror surface (5).

The shape of the perimeter of the protrusion (3) is describing a peripheral portion of a cylinder of equal shape as the inner circumference of the air duct (7) and has an equal or fraction larger outer diameter, thus enabling the protrusion (3) when inserted into the duct (7) to clamp into said air duct (7) and the protrusion further has a wall thickness (9) arranged in such a way that it contains the function of progressively narrowing and flattening the air outlet (7) in the direction of the estuary in order to faster and under downward pressure diverge the air over the mirror surface (5)

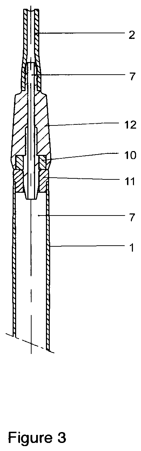

Figure 3 shows a longitudinal section of the handle (1) coupled with the connected air hose (9) that at its other rear end is connected to an air compressor, which mutual coupling is formed by two mutually magnetically attracted rings (10) and (11), of which one ring (10) is inextricably attached around the hose or around a tube part (12) that is connected to the hose (12) and the other ring (11) is positioned at the top, inextricably attached to the handle (1) and placed around the air duct (7) is disposed , in such a way that the rings in connected state connect the air duct (7) with the hose leak-proof through an attractive force, which is greater than the possible force exerted thereon during normal use of the mirror and smaller than above hand pull force, so that connection can be easily broken by pulling apart the hose and the handle by hand force.

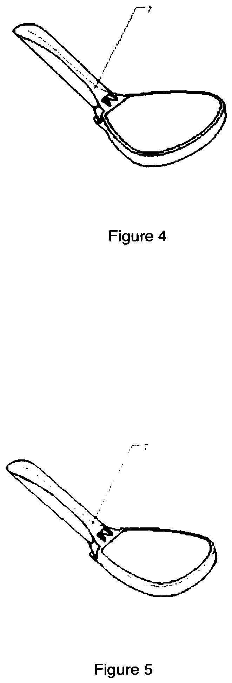

Figures 4 and 5 show two mirrors of different size and shape with a partial identically shaped part of protrusion , as far as it concerns the insertion in the air duct (7) and an different size and shape of the protrusion in so far as it concerns the control of the flow of air over the reflecting surface.

In order to facilitate the cleaning and sterilization ot the handle and to make possible use of the mirror with handle without air supply, the link between air supply and the handle is arranged in such a way that the air supply pipe contains a rigid end part that only is to be inserted into the air duct at the back of the handle to enable coupling through the mutual magnetically attracted rings of which one is inextricably attached along the rigid end part of the air supply and the other is inextricably attached onto the hollow handle, thus providing an air-tight connection and by which the air duct in the handle and the air supply tube are leak free connected with each other and by which the magnetic adhesive force is chosen in such a way that the coupling is maintained when a pulling force is applied in a situation that the handle is normally used and that is disconnected by means of a larger exertion hand force applied to it.

On the basis of Figures 1-5 , the invention will in a preferred embodiment be described in detail. Figure 1 shows a handle (1) in longitudinal crosssection with an air duct (7) and a flexible air supply pipe connected at the top (2) and inserted profusion at the bottom (3) connected with a mirror (4) with a hydrophobic and non-stick made reflecting surface (5) that is unlimited from the air outlet (6) of the air duct (7) to all directions of the reflecting surface (5) i.e. not limited by an upstanding casing or rim. Figure 2 shows the protrusion (3) standing on its own with the mirror (4) that is rigidly attached to it. At the upper side of the protrusion (3) either vertical walls or control surfaces (8) are formed, that have been arranged into such an angle with respect to the longitudinal direction (i.e. in the air flow direction) of the protrusion (3) and in such way that the air flowing along those surfaces is diverged congruent to the at the protrusion connected angle of the mirror surface (5).

The shape ot the perimeter ot the protrusion (3) is describing a peripheral portion of a cylinder of equal shape as the inner circumference of the air duct (7) and has an equal or fraction larger outer diameter, thus enabling the protrusion (3) when inserted into the duct (7) to clamp into said air duct (7) and the protrusion further has a wall thickness (9) arranged in such a way that it contains the function of progressively narrowing and flattening the air outlet (7) in the direction of the estuary in order to faster and under downward pressure diverge the air over the mirror surface (5) Figure 3 shows a longitudinal section of the handle (1) coupled with the connected air hose (9) that at its other rear end is connected to an air compressor, which mutual coupling is formed by two mutually magnetically attracted rings (10) and (11), of which one ring (10) is inextricably attached around the hose or around a tube part (12) that is connected to the hose (12) and the other ring (11) is positioned at the top, inextricably attached to the handle (1) and placed around the air duct (7) is disposed , in such a way that the rings in connected state connect the air duct (7) with the hose leak-proof through an attractive force, which is greater than the possible force exerted thereon during normal use of the mirror and smaller than above hand pull force, so that connection can be easily broken by pulling apart the hose and the handle by hand force.

Figures 4 and 5 show two mirrors of different size and shape with a partial identically shaped part of protrusion , as far as it concerns the insertion in the air duct (7) and an different size and shape of the protrusion in so far as it concerns the control of the flow of air over the reflecting surface.

The invention is defined in the following claims; other embodiments are merely exemplary and do not constitute a part of the invention.

## Claims

1. A mirror device for dental examination, comprising: a handle portion (1) and a mirror portion (9) with a reflective surface (5) for the purpose of said reflective surface being effectively free of moisture and dirt during use in a dental procedure and for the purpose of easier cleaning and sterilization of both said handle portion and said mirror portion for re-use in between other dental procedures, wherein:
said handle portion is hollow and contains an air duct (7) comprising at one end an air inlet opening (11) and on the other end an air outlet opening (6),
wherein said reflective surface contains a hydrophobic or super-hydrophobic, and a non-sticking material,
the device further comprising a detachable coupling (12) adapted to be coupled to a compressed air supply in order to supply an air flow through said air duct of said handle portion, with the purpose to divert said air flow over said reflecting surface, during use in said dental procedure;
the device being **characterised by** comprising:
a protrusion (3) that is inextricably attached to said mirror portion and shaped such, that said protrusion can be mounted detachably within said air outlet opening of said air duct of said handle portion by clamping, with the purpose to use said mirror device as a dental mirror in said dental procedure, wherein, in the detached state, said protrusion is from all sides accessible for cleaning and sterilization;
further **characterized in that**, for the purpose of diverting a controlled and customized air flow over said reflecting surface, both said air duct in said handle portion and said protrusion on said mirror portion are adapted to each other such, that said air duct is further shaped and narrowed by the presence of said protrusion, and consequently the shape of said protrusion is adapted to influence the free passage of compressed air through said narrowed air duct, causing the aforementioned effect of controlling and customizing said air flow just before said air outlet opening.

2. The mirror device according to claim 1, further comprising upstanding control surfaces (8) forming an integral part of said protrusion on said mirror portion and wherein said upstanding control surfaces are located just before the air outlet opening and are specifically shaped to further steer and guide said compressed air flow from within the space in the air duct, causing a downward and further diverging air flow over said reflective surface.

3. A mirror device according to claim 1 or 2, wherein the contour of said reflective surface is triangular and wherein, if a compressed air flow is led through the air duct (7) of said handle portion, said air flow is steered by upstanding control surfaces such, that the outflow of air will congruently and downwardly diverge over said triangular based surface in an angle congruent to said triangular based shape.

4. A mirror device according to any of the preceding claims, wherein said reflective surface can be created in a variety of distinct shapes and sizes and wherein said protrusion for each mirror portion of said variety fits within the air outlet duct of said handle portion and further **characterized in that** said protrusion is shaped such that, when said handle portion and said mirror portion are in attached configuration and connected to a compressed air supply, the shape of said custom protrusion is causing a further progressively narrowing and flattening and guidance of said air flow from within said air duct, causing said air flow to diverge downward over the reflective surface

5. A mirror device according to any of the preceding claims, wherein the coupling of said air inlet opening to an air hose (9) of the compressed air supply consists of two mutual magnetically attracted rings, one of which (10) is rigidly connected to the coupling (12) that is connected to said air hose (9) and the second ring (11) is rigidly mounted in said air inlet of said handle portion, resulting in a leak-proof connection between said handle portion and said coupling with air hose, whereby said magnetic attraction is chosen such, that said coupling is maintained at a normal use exerted traction and disconnected at greater exerted hand pull.

## Patentansprüche

1. Spiegelvorrichtung zur zahnärztlichen Untersuchung, umfassend:
einen Griffabschnitt (1) und einen Spiegelabschnitt (9) mit einer reflektierenden Oberfläche (5), mit der Absicht, die zuvor genannte reflektierende Oberfläche während der Verwendung in einem zahnärztlichen Eingriff effektiv frei zu halten von Feuchtigkeit und Schmutz, und wobei zum Zwecke einer einfacheren Reinigung und Sterilisation, sowohl der zuvor genannte Griffabschnitt als auch der zuvor genannte Spiegelabschnitt wiederverwendbar sind zwischen verschiedenen zahnärztlichen Eingriffen,
wobei der hervorgenannte Griffteil hohl ist und einen Luftkanal enthält, der an einem Ende eine Lufteintrittsöffnung (11) und am anderen Ende eine Luftaustrittsöffnung (6) aufweist, wobei die zuvor genannte reflektierende Oberfläche ein hydrophobes oder superhydrophobes und nichtklebendes Material enthält,
wobei außerdem die zuvor genannte Vorrichtung eine abnehmbare Kupplung (12) aufweist, die dazu ausgelegt ist mit einer Druckluftversorgung gekoppelt zu werden, um während des Gebrauchs einen Luftstrom durch den zuvor genannten Luftkanal oder zuvor genannten Griffabschnitt durchzuführen, mit dem Absicht, den zuvor genannten Luftstrom über die zuvor genannte reflektierende Oberfläche zu divergieren während zuvor genannten zahnärztlichen Eingriff;
wobei die zuvor genannte Vorrichtung **dadurch gekennzeichnet ist, dass** sie Folgendes umfasst:
einen Vorsprung (3), der untrennbar mit den zuvor genanntem Spiegelabschnitt verbunden und so geformt ist, dass der zuvor genannten Vorsprung innerhalb der zuvor genannten Luftaustrittsöffnung des zuvor genannten Luftkanals des zuvor gennanten Griffabschnitts durch Klemmen abnehmbar angebracht werden kann, um die zuvor genannte Spiegelvorrichtung als Mundspiegel zu verwenden in dem zuvor genannten zahnärztlichen Eingriff anzuwenden, in dem der zuvor genannte Vorsprung in getrennten Zustand von allen Seiten zum Reinigen und Sterilisieren zugänglich ist;
weiterhin **dadurch gekennzeichnet, dass** zum Zwecke der Divergenz eines gesteuerten und customisierten Luftstroms über die zuvor genannte reflektierende Oberfläche, wobei sowohl der zuvor genannte Luftkanal in dem zuvor genannten Griffabschnitt als auch der zuvor genannte Vorsprung an dem zuvor genannten Spiegelabschnitt auf solche Art in Form aufeinander abgestimmt sind, dass der zuvor genannte Luftkanal durch das Vorhandensein des zuvor genannten Vorsprungs weiter geformt und verengt wird und folglich an die Form des zuvor genannten Vorsprungs angepasst ist, damit den freien Durchgang oder die zuvor genannte Druckluft durch den verengten Luftkanal beeinflusst wird, was den zuvor genannten Effekt bewirkt und ermöglicht dass den zuvor genannten Luftstrom unmittelbar vor der zuvor genannten Luftaustrittsöffnung gesteuert und angepasst wird.

2. Spiegelvorrichtung nach Anspruch 1, ferner mit aufrechten Steuerflächen (8), die einen integralen Bestandteil des zuvor genannten Vorsprungs an dem zuvor genannten Spiegelabschnitt bilden, und wobei den zuvor genannten aufrechten Steuerflächen, die sich unmittelbar vor der zuvor genannten Luftauslassöffnung befinden und speziell geformt sind, um weiter zu lenken und zu führen wobei der zuvor genannten Druckluftstrom aus dem Raum in dem zuvor genannten Luftkanal kommt und einen abwärts gerichteten und weiter divergierenden Luftstrom über die zuvor genannte reflektierende Oberfläche verursacht.

3. Spiegel vorrichtung nach Anspruch 1 oder 2, bei der die Kontur oder Grundform von der zuvor genannte eflektierende Oberfläche dreieckig ist und wenn ein Druckluftstrom durch den zuvor genannten Luftkanal vom zuvor genannten Griffabschnitt geleitet wird, der zuvor genannte Luftstrom durch aufrechte Steuerflächen gesteuert wird, derart, daß der Luftaustritt kongruent und abwärts divergiert über die zuvor genannte dreieckige Grundform in einem Winkel, kongruent an der zuvor genannte dreieckigen Grundform.

4. Spiegelvorrichtung nach einem der vorhergehenden Ansprüche, wobei die zuvor genannte reflektierende Oberfläche in einer Vielzahl unterschiedlicher Formen und Größen erzeugt werden kann und der Schutz für jeden Spiegelabschnitt oder die Vielzahl in den zuvor genannten Luftauslasskanal vom zuvor genannten Griffabschnitt passt und wobei der zuvor genannte Vorsprung so geformt ist, dass, wenn der zuvor genannte Griffabschnitt und der zuvor genannte Spiegelabschnitt in zusammengestellter Konfiguration und mit einer Druckluftversorgung verbunden sind, die Form des kundenspezifischen Vorsprungs eine weitere fortschreitende Verengung und Abflachung und Führung des zuvor genannten Luftstroms von innen bewirkt wobei der zuvor genannte Luftkanal bewirkt, dass der zuvor genannte Luftstrom über die zuvor genannte eflektierende Oberfläche nach unten divergiert.

5. Spiegelvorrichtung nach einem der vorhergehenden Ansprüche, wobei die Verbindung der zuvor genannten Lufteinlassöffnung mit einem Luftschlauch (9) oder der Druckluftversorgung aus zwei gegenseitig magnetisch angezogenen Ringen besteht, von denen einer (10) starr mit dem zuvor genannte Kupplung (12) verbunden ist, die mit dem zuvor genannte Luftschlauch (9) verbunden ist und der zweite Ring (11) starr in dem zuvor genannten Lufteinlass oder dem zuvor genannten Griffabschnitt montiert ist, was zu einer lecksicheren Verbindung zwischen dem zuvor genannten Griffabshnitt und der zuvor genannte Kupplung mit dem zuvor genannten Luftschlauch führt, wobei die magnetische Anziehungskraft so gewählt ist, dass die zuvor genannte Verbindung bei normalem Gebrauch eine ausgeübte Traktion beibehält und bei größerem ausgeübten Handzug gelöst wird.

## Revendications

1. Un appareil miroir pour examen dentaire, comprenant:
une partie de manche (1) et une partie de miroir (9) avec une surface réfléchissante (5) pour l'objective que la surface réfléchissante soit efficacement exempte d'humidité et la saleté pendant utilisation danse une procédure dentaire et danse le but la facilitation du nettoyage et la stérilisation de à la fois dite partie manche et dite partie miroir pour une réutilisation entre un nombre des autres procédures dentaires,
ladite partie de manche est creuse et contient un conduit d'air comprenant à une extrémité à une ouverture d'entrée d'air (11) et à l'autre extrémité à une ouverture de sortie d'air (6) ladite dite surface réfléchissante contient une surface hydrophobe ou super-hydrophobe d'une surface non collante matériel,
le dispositif comprenant en outre un raccord détachable (12) adapté pour être couplé à une alimentation en air comprimé afin de fournir un flux d'air à travers ledit conduit d'air ou ledit partie de manche, dans le but de dévier ledit flux de l'air sur ladite surface réfléchissante, pendant l'utilisation dans ladite procédure dentaire;
le dispositif étant **caractérisé par**:
une saillie (3) qui est fixée d'une manière inextricable à ladite partie miroir et formée de telle sorte que ladite saillie pourrait être montée d'une manière amovible danse dite ouverture la sortie d'air ou ledit conduit d'air ou ladite partie du manche, par un form serrangable, en utilisant ledit dispositif miroir comme miroir dentaire pendant ledit dentaire procédure, et en outre, dans un état détaché, est accessible à tous les côtés pour le nettoyage et la stérilisation;
caractérisé en outre, dans le but de diverger un flux d'air contrôlé et customisé sur ladite surface réfléchissante, en ce que à la fois ledit conduit d'air dans ladite partie de manche et ladite saillie sur ladite partie de miroir sont adaptés, l'un à l'autre, de telle sorte que ledit conduit d'air est en outre conformé et rétréci par la présence de ladite saillie, et pour cet objective, la forme de ladite saillie est adaptée pour influencer le libre passage de l'air comprimé, à travers ledit conduit d'air rétréci, provoquant l'effet susmentionné ou contrôlant et customisant ledit flux d'air, juste avant ladite ouverture de sortie d'air.

2. Dispositif de miroir selon la revendication 1, comprenant en outre des surfaces de commande verticales (8) en faisant partie intégrante de ladite saillie sur ladite partie de miroir et lesdites surfaces de commande verticales sont situées juste avant l'ouverture de sortie d'air et sont spécifiquement conçues pour orienter et guider davantage ledit flux d'air comprimé provenant de l'intérieur de l'espace dans le conduit d'air, en provoquant un flux d'air divergent vers le bas et davantage sur ladite surface réfléchissante.

3. Dispositif à miroir selon la revendication 1 ou 2, dans lequel le contour ou ladite surface réfléchissante est triangulaire et si un flux d'air comprimé est conduit à travers le conduit d'air ou ladite partie de manche, ledit flux d'air est dirigé par des surfaces de commande verticales telles que, l'écoulement d'air divergera de façon congruente et descendante sur ladite surface à base triangulaire selon un angle congruemment avec ladite forme à base triangulaire.

4. Dispositif à miroir selon l'une quelconque des revendications précédentes, ladite dite surface réfléchissante peut être créée dans une variété de formes et de tailles distinctes et ladite dite saillie pour chaque partie de miroir ou ladite variété s'adapte à l'intérieur du conduit de sortie d'air de ladite partie de manche et encore **caracterisé en ce que** ladite saillie a une forme telle que, lorsque ladite partie de manche et ladite partie de miroir sont en configuration attachée et connectées à une alimentation en air comprimé, la forme de ladite saillie custumisé provoque un rétrécissement et aplatissement et un guidage progressive supplémentaire de ledit écoulement d'air de l'intérieur ledit conduit d'air, faisant diverger ledit flux d'air vers le bas à travers sur la surface réfléchissante

5. Dispositif à miroir selon l'une quelconque des revendications précédentes, le couplage de ladite ouverture d'entrée d'air à un tuyau à air (9) ou l'alimentation en air comprimé consiste en deux anneaux mutuellement attirés magnétiquement, dont l'un (10) est relié rigidement à la couplage (12) qui est connecté audit tuyau d'air (9) et la deuxième bague (11) est montée rigidement dans ladite entrée d'air ou ladite partie de manche, résultant en une connexion sans fuites entre ladite partie de manche et ledit couplage avec le tuyau d'air, en choisir ladite attraction magnétique de telle manière sorte que ledit couplage soit maintenu à une traction normale exercée et soit déconnecté à une traction manuel exercée plus importante.
